# EUROPEAN PATENT SPECIFICATION

(11) **EP 1 904 120 B1**
(45) Date of publication and mention of the grant of the patent: **28.04.2010**
(21) Application number: 05763218.4
(22) Date of filing: 20.07.2005
(51) Int. Cl.: A61M 1/16, B01F 15/04

(54) **An apparatus and process for on-line preparation of a medical liquid**
Gerät und Verfahren für die online-Herstellung einer medizinischen Flüssigkeit
Appareil et procédé pour la préparation en ligne d'un liquide medical

(43) Date of publication of application: 02.04.2008
(73) Proprietor: Gambro Lundia AB, 22010 Lund (SE)
(72) Inventor: ROSSI, Andrea, I-46020 San Giacomo delle Segnate (IT); FAVA, Massimo, I-41037 Mirandola (IT)
(74) Representative: Ponzellini, Gianmarco
(86) International application number: PCT/IB2005/002101
(87) International publication number: WO 2007/010316

(56) References cited:
- US-A- 5 344 231
- US-A- 5 833 949
- US-A1- 2005 040 110
- US-B1- 6 793 827

## Description

### Background of the Invention

The invention relates to an apparatus and a process for on-line preparation of a medical liquid.

Specifically, though not exclusively, the invention can be usefully applied in the on-line preparation of a dialysis liquid and/or a replacement liquid in a machine for treatment of renal insufficiency, such as for example a machine for performing one or more of the following treatments: hemodialysis, hemofiltration, hemodiafiltration.

US 5,344,231 describes an apparatus for preparation of a medical liquid, comprising: a main preparation line, a first injection line between an inlet of a first concentrate and a first point of injection into the main line, a second injection line between an inlet of a second concentrate and a second point of injection in the main line situated downstream of the first point of injection, a first conductivity sensor arranged in the main line between the first and the second point of injection, and a second conductivity sensor arranged in the main line after the second point of injection. The apparatus described in US 5,344,231 further comprises a first dosing pump of the first concentrate operating on the first injection line and controlled on the basis of signals emitted by the first conductivity sensor, and a second dosing pump of the second concentrate operating on the second injection line and controlled on the basis of signals emitted by the second conductivity sensor.

The dosing system known from US 5,344,231 can present a problem in the regulation of the second concentrate, i.e. the concentrate injected into the main line by the second dosing pump in a point downstream of a preceding point of injection of another concentrate. The particular problem occurs in a case in which the desired quantity of the second concentrate introduced into the main line is relatively small. In this case the change of conductivity of the medical liquid in the main preparation line downstream of the second point of injection, which variation is due to the introduction of the second concentrate, is relatively modest, with a consequent need for the second conductivity sensor to be especially precise in order to contain the dosing error within predetermined limits. In the specific case the problem is aggravated by the fact that the medical liquid in the main preparation line, immediately before reaching the second injection point, already exhibits a relatively high conductivity, as at the first point of injection, upstream of the second, a certain quantity of the first concentrate has already been injected. Consequently the second conductivity sensor must be able to measure relatively high conductivity values with an extremely small margin of error. In the case of injection of a modest quantity of the second concentrate, if relatively high dosage percentage errors are to be avoided in the second concentrate, the second conductivity sensor must be sophisticated and expensive.

It is also known from document US 6793827 an apparatus for on-line preparation of a medical liquid having a main line for preparation of the medical liquid connected to an inlet of a liquid and having at least a first injection point of a concentrate. A first injection circuit is connected to an inlet of a first concentrate and to the first injection point.

A first sensor is predisposed to emit a signal indicating a chemical-physical property of the liquid situated in the first injection circuit and a control unit is prediposed for controlling a first pump on the basis of the signal emitted by the first sensor.

### Summary of the invention

A main aim present invention is to provide a process for preparation of a medical liquid which is able to obviate the described drawback in the prior art.

A further aim of the invention is to provide an apparatus which is able to actuate the above-mentioned process.

An advantage of the invention is that is provides a process and an apparatus for on-line preparation of a medical liquid which are very precise in the dosage of the liquid components.

A further advantage of the invention is that is makes available a process and an apparatus which are particularly precise, even in cases of injections of various concentrates at several points in a main preparation line.

A still further advantage is that it gives rise to a process and an apparatus which are simple and economical to realise.

These aims and advantages and others besides are all achieved by the present invention as it is characterised in one or more of the appended claims.

In a first embodiment of the invention, an apparatus for the on-line preparation of a medical liquid comprises: a main preparation line of a medical liquid having at least a first injection point of a concentrate; a first injection circuit connected to an Inlet of a first concentrate and to the first injection point; a sensor for emitting a signal indicating a chemical-physical property of the liquid in the first injection circuit before the first injection point; a first pump and a second pump for the circulation of liquids in the first injection circuit; a control unit predisposed for controlling one of the pumps on the basis of a signal emitted by the sensor, and for controlling the other of the pumps on the basis of a predetermined flow-rate.

In a further embodiment, the apparatus of the first embodiment comprises a second injection point of a second concentrate in the main line which is located before the first injection point, with reference to the liquid circulation direction in the main line.

In a further embodiment, the first pump of one of the preceding embodiments is arranged between the inlet of a first concentrate and a point of dilution of the first injection circuit in which the first concentrate is diluted.

In a further embodiment, in the apparatus of the preceding embodiment a control unit is predisposed to control the first pump on the basis of the signal of a chemical-physical property.

In a further embodiment, in the apparatus of any one of the preceding embodiments, one of the pumps is arranged between a dilution point of the first injection circuit, in which the first concentrate is diluted, and the first injection point.

In a further embodiment, in the apparatus of any one of the preceding embodiments, one of the pumps is arranged in a dilution line which terminates at dilution point in the first injection circuit in which the first concentrate is diluted.

In a further embodiment, the apparatus of any one of the preceding embodiments comprises a second sensor which is able to emit a signal indicating a fluid flow-rate in a tract of the first injection circuit in which one of the pumps operates, the control unit being predisposed to control one of the pumps on the basis of the flow-rate signal.

In a further embodiment, in the apparatus of the preceding embodiment the second flow rate sensor comprises a sensor for detecting an operative velocity of the pump operating in the tract of circuit, or in a flow meter operating in the tract of circuit.

In a further embodiment, in the apparatus of any one of the preceding embodiments the control unit is predisposed to control one or more of the pumps at a constant operative velocity, the indicative value of a flow rate being a desired value of the operative velocity.

In a further embodiment, an apparatus for on-line preparation of a medical liquid comprises: a main preparation line of a medical liquid having at least a first injection point of a concentrate; a first injection circuit connected to an inlet of a first concentrate and to the first injection point; a sensor for emitting a signal indicating a chemical-physical property of the liquid in the first injection circuit upstream of the first injection point; at least a pump for circulation of at least a liquid in the first injection circuit; a second injection circuit connected to an inlet of a second concentrate and to a second point of injection of concentrate arranged along the main line upstream of the first injection point; a control unit predisposed to control the pump on the basis of the signal emitted by the sensor.

In a further embodiment, the apparatus of the preceding embodiment comprises a further pump for the circulation of liquid in the first injection circuit, the control unit being predisposed to control the further pump on the basis of a value indicating a flow rate in a tract of the first injection circuit.

In a further embodiment, in the apparatus of any of the preceding embodiments, the first injection circuit comprises a first injection line connected to the inlet of a first concentrate and to the first injection point, and a dilution line terminating in a dilution point in the first injection line.

In a further embodiment, in the apparatus of the preceding embodiment, the dilution line is predisposed for circulation of a liquid and for not inducing substantial increases in ionic concentration of the liquid.

In a further embodiment, in the apparatus of one of the preceding embodiments, the dilution line is connected to a branch point situated in the main line.

In a further embodiment, in the apparatus of the preceding embodiment, the branch point is arranged upstream of the first injection point, with reference to the liquid circulation direction in the main line.

In a further embodiment, in the apparatus of one of the two preceding embodiments, the branch point is arranged before the second injection point of a second concentrate in the main line.

In a further embodiment, in an apparatus of any one of the preceding five embodiments, the sensor of a chemical-physical property of the liquid operates between the point of dilution and the first injection point.

In a further embodiment, in an apparatus according to any one of the preceding embodiments, the main line is arranged between an inlet of a liquid (water) and a connection for the attaching of a blood treatment device (dialyzer).

In a further embodiment, an apparatus of any one of the preceding embodiments comprises a fluid balance unit and a sterile filter arranged on the main line after the first injection point.

In a further embodiment, an apparatus according to any one of the preceding embodiments comprises a main supply pump arranged after the first injection point for the circulation of liquid in the main line.

In a further embodiment, in an apparatus according to any one of the preceding embodiments, the inlet of a first concentrate comprises a connection for a liquid container.

In a further embodiment, an apparatus of any one of the preceding embodiments comprises a container of a liquid concentrate connected to the first injection circuit.

In a further embodiment, in an apparatus according to any one of the preceding embodiments, the sensor of a chemical-physical property of the liquid comprises at least an ionic concentration sensor.

In a further embodiment, in an apparatus according to any one of the preceding embodiments, the sensor of an ionic concentration comprises a sensor of electrical conductibility.

In a further embodiment, a machine for extracorporeal treatment of blood comprises an apparatus for on-line preparation of a medical liquid realised according to any one of the preceding embodiments.

In a further embodiment, a machine according to the preceding embodiment is predisposed to perform one or more of the following treatments: hemodialysis, hemofiltration, hemodiafiltration.

In a further embodiment, a process for on-line preparation of a medical liquid comprises stages of: circulating a liquid in a main preparation line having at least a first injection point; injecting at least a first concentrate from a first injection circuit into the first injection point; measuring at least a value of a chemical-physical property of a liquid flowing in the first injection circuit; controlling at least a first flow rate of liquid flowing in the first injection circuit.on the basis of the value measured of a chemical-physical property; controlling at least a second flow rate of a liquid flowing in the first injection circuit on the basis of a predetermined flow-rate value.

In a further embodiment, a process according to the preceding embodiment comprises a stage of supplying a dilution liquid at a dilution point of the first injection circuit in order to reduce an ionic concentration of the first concentrate.

In a further embodiment, in a process according to the preceding embodiment the dilution liquid is removed from a branch point in the main line.

In a further embodiment, in a process according to one of the two preceding embodiments, the value of a chemical-physical property is measured downstream of the point of dilution.

In a further embodiment, in a process according to any one of the four preceding embodiments, the first flow rate flows into a tract of circuit comprised between a source of the first concentrate and a point of dilution of the first concentrate, or into a tract of circuit comprised between the point of dilution and first injection point, or into a tract of circuit upstream of the point of dilution, where the dilution liquid flows.

In a further embodiment, in a process of any one of the five preceding embodiments, the second flow rate flows in a tract of circuit comprised between a source of the first concentrate and a point of dilution of the first concentrate, or in a tract of circuit comprised between the point of dilution and the first injection point, or in a tract of circuit upstream of the point of dilution where a dilution liquid flows.

In a further embodiment, in a process according to any one of the six preceding embodiments, the second flow rate is controlled at a constant value over at least a predetermined time period.

In a further embodiment, in a process according to the preceding embodiment, the value of a chemical-physical property is measured during the predetermined time period.

In a further embodiment, a process according to any one of the eight preceding embodiments comprises a stage of injecting at least a second concentrate in a second injection point arranged in the main line before the first injection point.

In a further embodiment, in a process according to any one of the nine preceding embodiments, the main line takes liquid from a liquid source and sends liquid to a. blood treatment device.

In a further embodiment, in a process according to any one of the ten preceding embodiments the first concentrate is removed from a liquid container.

In a further embodiment, in a process of any one of the eleven preceding embodiments, the chemical-physical property indicates the ionic concentration.

In a further embodiment, in a process according to the preceding embodiment, the above property indicating the ionic concentration is the electrical conductibility.

In a further embodiment, in a process of any one of the thirteen preceding embodiments, the medical liquid is a sterile infusion liquid in an extracorporeal blood circuit, or is a dialysis liquid.

In a further embodiment, a process for on-line preparation of a medical liquid comprises stages of: circulating a liquid in a main preparation line; circulating at least a first concentrate in a first injection circuit towards a first injection point in the main line; circulation of at least a second concentrate in a second injection circuit towards a second injection point situated in the main line upstream of the first injection point; measuring at least a value of a chemical-physical property of a liquid flowing in the first injection circuit; controlling at least a first flow rate of a liquid flowing in the first injection circuit on the basis of the value measured of a chemical-physical property.

In an embodiment of the invention, an injection circuit of a concentrate comprises at least two flows of liquids having different ionic concentrations, in which one of the two flows is controlled by a signal indicating an ionic concentration at a point of the injection circuit, while the other of the two flows is controlled on the basis of a predetermined flow rate. The predetermined flow rate can be pre-set, in which case a calculation is made of a value of ionic concentration in the concentrate for obtaining the desired dosage, or the calculation can be made once the ionic concentration has been preset. The preset flow rate value can vary, not only in the desired dosage and the ionic concentration of the concentrate, but also in the flow rate in the main line of preparation of the medical liquid.

In an embodiment of the invention, an injection circuit of a concentrate comprises a dilution line which takes a part of the flow from a main liquid preparation line to an injection line of the concentrate. The flow in the dilution line does not increase substantially in its ionic concentration. It is therefore possible to use the flow in the dilution line, which can have a very small concentration (for example in the case of a flow of deionized water) to reduce the ionic concentration of the flow in the injection line and thus facilitate the measurement of the ionic concentration in the injection line.

In an embodiment of the invention, a preparation line of medical liquid comprises at least two concentrate injection points, in which the injection point located more downstream is connected to an injection circuit provided with at least a flow controlled on the basis of one or more parameters which are characteristic of the circuit, such as for example a flow rate in the circuit or a chemical-physical characteristic (for example ionic concentration or another parameter indicating the concentration) of the liquid forming the injection flow.

In an embodiment of the invention, the conductibility of the liquid which is injected at a point of the preparation line, and the flow rate of the liquid itself, are controlled independently of each other. The independent control can be made by controlling two different actuators (for example two pumps) operating on the circuit.

Further characteristics and advantages of the present invention will better emerge from the detailed description that follows of at least an embodiment of the invention, illustrated by way of non-limiting example in the figure of the drawing.

### Brief Description of the Drawings

The description will now be made with reference to the drawing, which is included by way of non-limiting example, and in which:

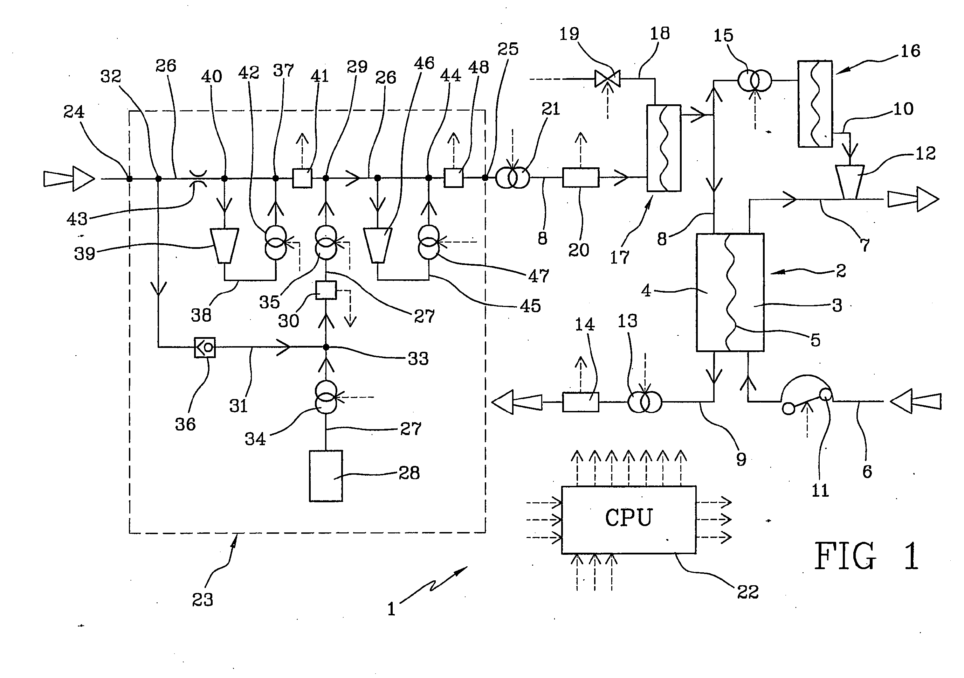

- Figure 1 is a diagram of an apparatus for preparation of a medical liquid according to the present invention.

### Detailed Description

With reference to figure 1, 1 denotes in its entirety a hemodiafiltration apparatus for treatment of renal insufficiency.

The hemodiafiltration apparatus 1 comprises a blood treatment device 2 having a blood chamber 3 and a fluid chamber 4 that are separated one from another by a semipermeable membrane 5. A blood removal line is denoted by 6, and connects a patient's vascular access with an inlet of the blood chamber 3; 7 denotes a blood return line which connects an outlet of the blood chamber 3 with the patient's blood vessel system, and 8 denotes a dialysis fluid supply to an inlet of the fluid chamber 4. 9 denotes a discharge line connecting an outlet of the fluid chamber 4 with a discharge, while 10 indicates an infusion line for directly introducing a replacement liquid into the patient's blood. In the specific case the infusion line 10 introduces the replacement liquid into the extracorporeal blood circuit, in particular into the return line 7, downstream of the blood treatment line 2 (post-infusion). An infusion line can also be provided which introduces the replacement liquid into the removal line 6 (pre-infusion), or into the removal line 6 and the return line 7 (combination of pre- and post-infusion), or directly into the intracorporeal circuit of the patient through a vascular access. The infusion line 10 is a branch of the dialysis fluid supply line 8.

11 denotes a blood pump for circulation of blood in the extracorporeal circuit, 12 a blood chamber arranged in the extracorporeal circuit, 13 a discharge pump for the circulation of the discharge fluid exiting from the fluid chamber 4 along the discharge line 9, 14 a sensor for detecting the flow rate of the discharge fluid along the discharge line 9, 15 an infusion pump for the circulation of the replacement fluid along the infusion line 10, 16 a filter for purifying the replacement fluid along an infusion line 10, 17 a filter for purifying the dialysis liquid along the supply line 8 upstream of the infusion line 10, 18 a rinsing line connected to an outlet of a non-filtered chamber of the filter 17 of the dialysis fluid 17, 19 an intercept valve arranged on the rinsing line and commanded to open intermittently for periodic rinsing of the non-filtered chamber of the filter 17. 20 denotes a fresh dialysis fluid flow rate sensor along the supply line 8 upstream of the infusion line 10, 21 a pump for circulating the dialysis fluid arranged along the supply line 8. The flow rate sensors 14 and 20 collaborate, in a known way, commanded by a control unit 22, in controlling the fluid balance of the patient subjected to the extracorporeal blood treatment, which also signifies the patient's weight drop.

The hemodiafiltration apparatus 1 can also be used as a dialysis apparatus, without an infusion flow along the infusion line 10, or can be used as a hemodiafiltration apparatus, without dialysis liquid flow to the blood treatment device 2.

23 denotes, in its entirety, an apparatus for preparation of a fresh dialysis fluid line made according to the present invention.

The apparatus 23 has an inlet 24 for receiving a water flow, and an outlet 25 for providing a fresh dialysis liquid flow. The outlet 25 is connected to the supply line 8.

The preparation apparatus 23 comprises a main preparation line 26 which extends from the inlet 24 to the outlet 25. The main line 26 and the supply line 8 form a single liquid circulation line which extends internally of the hemodiafiltration apparatus 1 between the inlet 24 and a connection for the inlet of the fluid chamber 4 of the blood treatment device 2.

A first injection line 27 extends between an inlet of a concentrate 28 and a first injection point 29 arranged on the main line 26. In this embodiment the inlet 28 of the first concentrate comprises a connection for a bag of liquid. The protection apparatus 23 can also comprise the bag of liquid connected to the first injection line 27. The first concentrate can be in a different form, for example salts contained in a cartridge destined to be crossed by a liquid.

A sensor 30 is predisposed on the injection line 27 for emitting a signal to indicate a chemical-physical property of the liquid in the first injection Inie 27. In this embodiment the sensor 30 indicates the ionic concentration of the liquid, such as for example an electrical conductibility sensor.

The control unit 22 is predisposed to control the dosage of concentrate at the first injection point 29 on the basis of the signal provided by the sensor 30.

A dilution line 31 extends between a branch point 32 of the main line 26 and a dilution point 33 in the first injection line 27.

The dilution line 31 is structured and arranged so as to be crossed by a liquid (the liquid at the inlet 24 which in this embodiment is water) and not to induce substantial modifications to the above-mentioned chemical-physical property (in this embodiment the ionic concentration) in the liquid.

The conductivity sensor 30 operates between the dilution point 33 and the first injection point 29.

The branch point 32 is arranged before the first injection point 29, with reference to the advancement of the liquid along the main line 26.

The preparation apparatus 23 comprises a device for dosing the concentrate introduced in the main line 26 through the first injection line 27. The dosing device comprises a first injection pump 34 arranged in the first injection line 27, between the inlet of the concentrate 28 and the dilution point 33 in the first injection line 27. The control unit 22 is predisposed to control the first injection pump 34 on the basis of the conductivity signal sent by the sensor 30.

The dosing device comprises a second injection pump 35 arranged in the first injection line 27, between the dilution point 33 and the first injection point 29. The second injection pump 35 is controlled on the basis of a desired flow rate value.

In a second embodiment of the invention, not illustrated, the second injection pump is arranged on the dilution line 31, between the branch point 32 and the dilution point 33.

A flow rate sensor is predisposed to emit a signal indicating a flow rate of fluid in the line where the second injection pump 35 is operating. The flow rate sensor can comprise a sensor for detecting an operative velocity of the second injection pump 35, or a flow meter operating in the line (27 or 31) where the second injection pump 35 is located.

The control unit 22 is predisposed to control the second injection pump 35 on the basis of a signal emitted by the second flow rate sensor. In this embodiment the control unit 22 controls the second injection pump 35 at a constant operating speed.

The control unit can control the second injection pump 35 on the basis of the conductivity signal supplied by the sensor 30 and the first injection pump 34 on the basis of a desired flow rate. In this case a flow rate sensor is provided which is able to emit a signal indicating a flow rate of the first injection pump 34. The first flow rate sensor can comprise a sensor for detecting an operative velocity of the first injection pump 34, or a flow meter operating in the tract of line 27 where the first injection pump is located. The control unit 22 will be predisposed in this case for controlling the first injection pump 34 on the basis of the first flow rate signal, for example for controlling the first injection pump 34 at a constant operating speed for delivering a liquid flow rate at a known constant value.

The dilution line 31 is provided with a non-return valve 36 which prevents flow from the dilution point 33 towards the branch point 32.

The main line 26 comprises a second injection point 37 of a concentrate arranged after the branch point 32 and before the first injection point 29. A second injection line 38 extends between a source 39 of a second concentrate and the second injection point 37. In this embodiment the source of the second concentrate 39 is in the form of a cartridge of concentrate in powder or granule form which receives the liquid coming from a branch point 40 of the main line 26 situated upstream of the injection point 29. A conductivity sensor 41 is arranged on the preparation line 26 downstream of the second injection point 37. A circulation pump 42 of the second concentrate, arranged along the second injection line 38, is controlled on the basis of the conductivity signal of the second sensor 41.

A third injection point 44 of a third concentrate is arranged downstream of the first injection point 29. 45 denotes a third injection line, 46 a source of a third concentrate (for example in the form of powders or granules in a cartridge), 47 an injection pump controlled by a conductivity signal provided by a sensor 48 located on the preparation line 26 downstream of the third injection point 44.

The preparation apparatus 23 of the dialysis fluid is as follows.

The water, possibly already processed (for example purified, degassed and heated) enters the apparatus 23 through the inlet 24. The pump 21 circulates the liquid in the line formed by the line 26 and the line 8. The liquid flow rate at the outlet 25 of the apparatus 25 is measured by the sensor 20.

A part of the flow rate of water is diverted along the branch line 31 towards the first injection line 27. Here the pump 34 circulates a first concentrate coming from the concentrate 28 source. At the point of dilution 33 a mixing of the concentrate with the water coming from the line 31 is performed, having the effect of reducing the ionic concentration of the concentrate coming from the source 28. The ionic concentration of the mixture obtained is measured by the sensor 30. The control unit 22 compares the indicative value of the ionic concentration provided by the sensor 30, compares it with a set value and thus controls the pump 34 on the basis of this comparison, so that the measured value will tend to coincide with the set value. The pump 35 is commanded to supply a desired flow rate, for example a known constant flow. In this way the dosage of the injected liquid at the injection point 29 is realised with precision, as both the flow and the ionic concentration of the liquid introduced at the injection point 29 are known. The ionic concentration of the liquid injected at point 29 is known with a relatively small error percentage, without having to use a conductibility sensor 30 that is especially refined and expensive, as the value of conductibility measured is relatively high. The dilution line 31 is essentially used for reducing the measured conductibility value, which would otherwise be too high, with a consequent risk of imprecision in the measurements.

The dosing of the first concentrate in the main line 26 depends essentially on the values of two parameters of the concentrate at the injection point 29; the value of the ionic concentration of the injected concentrate, which is measured by the conductivity sensor 30, and the value of the flow rate of the injected concentrate, which can be calculated by the operative velocity of the second injection pump 35. Once the value of the flow rate flowing in the main line 26 is known (known thanks, for example, to the flow meter 20), dosage of the first concentrate is possible by regulating one or both of these values: it is possible, for example, to select a desired value for the flow rate of the pump 35, after which the ionic concentration value of the concentrate is calculated by virtue of which the quantity of concentrate injected corresponds to the desired dosage, thus the pump 34 is controlled by the signal supplied by the sensor 30, so that the concentrate has the above value calculated, while the pump 35 is controlled so as to respect the above desired flow rate value; to vary the concentrate diosage the flow rate, concentration or both can be changed. In theory for each main flow rate value along the line 26, infinite pairs of values of concentration of concentrate exist, satisfying a required dosage.

It is worth noting that with the described solution the dosage error caused by the imprecision (usually in the order of a few percent) in the bag concentration (source 28) containing the liquid concentrate is eliminated, as normally supplied by the producer. This error is not influential in this case, due to the fact that the concentrate conductibility injected at the injection point 29 and the concentrate flow rate are regulated independently of each other, by means of controlling two different pumps 34 and 35, one (34 or 35) controlled on the basis of a signal of conductivity, the other (35 or 34) on the basis of a flow rate signal. In other words, any error in the concentrate concentration in the bag provided by the producer and used as a concentrate source, any error in the concentration of the first concentrate in the dialysis liquid supplied by the apparatus 23 does not depend on the error of the bag producer, but essentially depends on the error in conductibility supplied by the sensor 30 and the flow rate error due to the pump 35, apart from the flow rate error due to the supply pump 21.

### Legend

- 1: hemodiafiltration apparatus
- 2: blood treatment device
- 3: blood chamber
- 4: fluid chamber
- 5: semipermeable membrane
- 6: blood removal line
- 7: blood return line
- 8: dialysis fluid supply line to the fluid chamber
- 9: fluid chamber discharge line
- 10: infusion line
- 11: blood pump
- 12: blood chamber
- 13: discharge pump
- 14: flow rate sensor
- 15: infusion pump
- 16: sterile filter of the replacement fluid
- 17: sterile filter of the dialysis fluid
- 18: filter 17 rinse line
- 19: intercept valve
- 20: flow rate sensor
- 21: dialysis fluid circulation pump
- 22: control unit
- 23: apparatus for on-line preparation of the dialysis fluid
- 24: water inlet
- 25: fresh dialysis fluid outlet
- 26: main preparation line
- 27: first concentrate injection line
- 28: first concentrate inlet
- 29: first concentrate injection point
- 30: conductivity sensor
- 31: dilution line
- 32: branch point
- 33: dilution point
- 34: first injection pump
- 35: second injection pump
- 36: non return valve
- 37: second concentrate injection point
- 38: second concentrate injection line
- 39: source of second concentrate
- 40: branch point
- 41: conductivity sensor
- 42: second concentrate circulation pump
- 43: choke section
- 44: third concentrate injection point
- 45: rhird concentrate injection line
- 46: source of third concentrate
- 47: third concentrate injection pump
- 48: conductivity sensor

## Claims

1. An apparatus for on-line preparation of a medical liquid, comprising:
a main line (26) for preparation of the medical liquid connected to an inlet (24) of a liquid and having at least a first injection point (29) of a concentrate;
a first injection circuit (27, 28) connected to an inlet of a first concentrate (28) and to the first injection point (29),
a first sensor (30) predisposed to emit a signal indicating a chemical-physical property of the liquid situated in the first injection circuit (27)
a control unit (22) prediposed for controlling a first pump (34) on the basis of the signal emitted by the first sensor (30), **characterized in that**
the first injection circuit has a dilution point (33) situated between the inlet of the first concentrate (28) and the first injection point (29); and **characterized in that** the apparatus further comprises:
a dilution line (31) which carries a dilution liquid to the dilution point (33) at which the first concentrate is diluted;
the first pump (34) and a second pump (35) for the circulation of liquids in the first injection circuit (27, 28) and in the dilution line (31); the first sensor (30) being predisposed to emit a signal indicating a chemical-physical property of the liquid situated in the first injection circuit (27) between the dilution point (33) and the first injection point (29).

2. The apparatus of claim 1, wherein the control unit (22) is predisposed to control the second pump (35) on the basis of a value indicating a preset flow rate.

3. The apparatus of claim 2, comprising a second sensor able to emit a signal indicating a fluid flow rate in a tract where the second pump (35) is operating, the control unit (22) being predisposed to control the second pump (35) on the basis of the signal emitted by the second sensor.

4. The apparatus of claim 3, wherein the second sensor comprises either a sensor for detecting an operative velocity of the second pump (35) or a flow meter for measuring the fluid flow rate in the tract in which the second pump (35) is operating.

5. The apparatus of any one of the preceding claims, wherein the first pump (34) is arranged in a location selected in the group consisting of the following three locations: between the inlet of the first concentrate (28) and the point of dilution (33), between the point of dilution (33) and the first injection point (29), and in the dilution line (31); the second pump (35) being arranged in one from among the above three locations but in a different location with respect to the first pump (34).

6. The apparatus of any one of the preceding claims, wherein the dilution line (31) has an inlet end which is connected to a branch point (32) situated in the main line (26) upstream of the first injection pump (29), with reference to a circulation direction of the liquid in the main line (26), and is predisposed in order not to induce substantial changes to an ionic concentration of the dilution liquid flowing from the inlet (32) end to the point of dilution (33).

7. The apparatus of any one of the preceding claims, wherein the main line (26) extends between the inlet (24) of a liquid, for example water, and a connection for connecting to a blood treatment device (2), for example a dialyzer.

8. The apparatus of any one of the preceding claims, wherein the first sensor (30) comprises at least a sensor which indicates an ionic concentration of a liquid, such as for example an electric conductibility sensor.

9. A machine for extracorporeal blood treatment, comprising an apparatus for on-line preparation of a medical liquid (23), realized according to any one of the preceding claims.

10. A process for on-line preparation of a medical liquid, comprising stages of:
circulating a liquid in a main preparation line (26) having at least a first injection point (29) of a concentrate;
injecting at least a first concentrate from a first injection circuit (27, 28) at the first injection point (29); characterizaed in that it further comprises the steps of:
injecting a dilution liquid from a dilution line (31) at a point of dilution (33) of the first injection circuit (27) for reducing an ionic concentration of the first concentrate before reaching the first injection point (29);
measuring at least a value of a chemical-physical property of the diluted first concentrate which flows in the first injection circuit (27, 28) after the point of dilution (33);
controlling at least a first flow rate of a liquid flowing in the first injection circuit (27, 28) or in the dilution line (31) on a basis of a value measured of a chemical-physical property;
controlling at least a second flow rate of a liquid flowing in the first injection circuit (27, 28) or in the dilution line (31) on a basis of a preset flow value.

11. The process of claim 10, wherein the dilution liquid is removed from a branch point (32) in the main line (26) and does not change an ionic concentration thereof between the branch point (32) and the point of dilution (33).

12. The process of claim 10 or 11, wherein the first flow runs in a tract of circuit selected in the group consisting of the following tracts of circuit: a tract of circuit comprised between a source of the first concentrate (28) and the point of dilution (33); a tract of circuit comprised between the point of dilution (33) and the first injection point (29); and a tract of circuit upstream of the point of dilution (33) where the dilution liquid flows; the second flow running in one of the above tracts of circuit but in a different tract from the tract in which the first flow runs.

13. The process of any one of claims from 10 to 12, wherein the main line (26) receives a first liquid, for example water, from a liquid source (24), and sends a second liquid, for example a dialysis liquid, to a blood treatment device (2).

14. The process of any one of claims from 10 to 13, wherein the chemical-physical property is electrical conductibility or another property indicating ionic concentration.

## Patentansprüche

1. Vorrichtung zur On-line-Herstellung einer medizinischen Flüssigkeit, umfassend:
eine Hauptleitung (26) zur Vorbereitung der medizinischen Flüssigkeit, die mit einem Einlauf (24) für eine Flüssigkeit verbunden ist und mindestens eine erste Injektionsstelle (29) für ein Konzentrat besitzt;
einen ersten Injektionskreislauf (27, 28), der mit einem Einlauf für ein erstes Konzentrat (28) und mit der ersten Injektionsstelle (29) verbunden ist,
einen ersten Sensor (30) zur Ausgabe eines Signals, das eine chemische-physikalische Eigenschaft der im ersten Injektionskreislauf (27) enthaltenen Flüssigkeit angibt,
eine Steuereinheit (22) zur Steuerung einer ersten Pumpe (34) abhängig von dem vom ersten Sensor (30) ausgegebenen Signal, **dadurch gekennzeichnet, daß**
der erste Injektionskreislauf eine Verdünnungsstelle (33) besitzt, die zwischen dem Einlauf für das erste Konzentrat (28) und der ersten Injektionsstelle (29) liegt; und **dadurch gekennzeichnet, daß** die Vorrichtung folgendes weiter umfasst:
eine Verdünnungsleitung (31), die eine Verdünnungsflüssigkeit zur Verdünnungsstelle (33) fördert, wo das erste Konzentrat verdünnt wird;
die erste Pumpe (34) und eine zweite Pumpe (35) zur Zirkulation von Flüssigkeiten im ersten Injektionskreislauf (27, 28) und in der Verdünnungsteitung (31); wobei der erste Sensor (30) zur Ausgabe eines Signals vorgesehen ist, das eine chemische-physikalische Eigenschaft der Flüssigkeit angibt, die im ersten Injektionskreislauf (27) zwischen der Verdünnungsstelle (33) und der ersten Injektionsstelle (29) enthalten ist.

2. Vorrichtung nach Anspruch 1, worin die Steuereinheit (22) zur Steuerung der zweiten Pumpe (35) abhängig von einem eine vorgegebene Durchflussmenge angebende Wert vorgesehen ist.

3. Vorrichtung nach Anspruch 2, umfassend einen zweiten Sensor zur Ausgabe eines Signals, das eine Flüssigkeitsdurchfluss in einem Abschnitt angibt, wo die zweite Pumpe (35) arbeitet, wobei die Steuereinheit (22) zur Steuerung der zweiten Pumpe (35) abhängig von dem vom zweiten Sensor ausgegebenen Signal vorgesehen ist.

4. Vorrichtung nach Anspruch 3, worin der zweite Sensor entweder einen Sensor zur Erfassung einer Betriebsgeschwindigkeit der zweiten Pumpe (35) oder einen Durchflussmesser zur Messung der Flüssigkeitsdurchfluss im Abschnitt, wo der zweite Pumpe (35) arbeitet, umfasst.

5. Vorrichtung nach irgendeinem der vorherigen Ansprüche, worin die erste Pumpe (34) sich in einem Bereich befindet, ausgewählt aus der Gruppe umfassend die folgenden drei Bereiche: zwischen dem Einlauf für das erste Konzentrat (28) und der Verdünnungsstelle (33), zwischen der Verdünnungsstelle (33) und der ersten Injektionsstelle (29), und in der Verdünnungsleitung (31); wobei die zweite Pumpe (35) in einem der oben genannten drei Bereiche aber in einem anderen Bereich als die erste Pumpe (34) angeordnet ist.

6. Vorrichtung nach irgendeinem der vorherigen Ansprüche, worin die verdünnungsleitung (31) ein Einlaufende besitzt, das mit einer Verzweigungsstelle (32) verbunden ist, die in der Hauptleitung (26) aufwärts von der ersten Injektionspumpe (29) in Bezug auf eine Zirkulationsrichtung der Flüssigkeit in der Hauptleitung (26) liegt, und das vorgesehen ist, um keine wesentliche Änderungen zu einer Ionkonzentration der vom Einlaufende (32) zur Verdünnungsstelle (33) strömenden Verdünnungsflüssigkeit zu verursachen.

7. Vorrichtung nach irgendeinem der vorherigen Ansprüche, worin die Hauptleitung (26) sich zwischen dem Einlauf (24) für eine Flüssigkeit, z.B. Wasser, und einem Anschluß zur Verbindung mit einer Blutbehandlungsvorrichtung (2), z.B. einem Dialysator, erstreckt.

8. Vorrichtung nach irgendeinem der vorherigen Ansprüche, worin der erste Sensor (30) mindestens einen Sensor umfasst, der eine Ionkonzentration einer Flüssigkeit, z.B. einen elektrischen Leitfähigkeitssensor, umfasst.

9. Maschine zur extrakorporalen Blutbehandlung, umfassend eine Vorrichtung zur On-line-Herstellung einer medizinischen Flüssigkeit (23) nach irgendeinem der vorherigen Ansprüche.

10. Verfahren zur On-line-Vorbereitung einer medizinischen Flüssigkeit, umfassend die folgenden Schritte;
Zirkulation einer Flüssigkeit in einer Hauptvorbereitungsleitung (26) mit mindestens einer ersten Injektionsstelle (29) für ein Konzentrat;
Injektion mindestens eines ersten Konzentrats aus einem ersten Injektionskreislauf (27, 28) an der ersten Injektionsstelle (29); **dadurch gekennzeichnet, daß** es die folgenden Schritte weiter umfasst:
Injektion einer verdünnungsflüssigkeit aus einer Verdünnungsleitung (31) an einer Verdünnungsstelle (33) des ersten Injektionskreislaufes (27) zur Reduktion einer lonkonzentration des ersten Konzentrats, bevor die erste Injektionsstelle (29) erreicht wird;
Messung mindestens eines Wertes einer chemischen-physikalischen Eigenschaft des verdünnten ersten Konzentrats, das im ersten Injektionskreislauf (27, 28) nach der Verdünnungsstelle (33) strömt;
Steuerung mindestens einer ersten Durchflussmenge einer im ersten Injektionskreislauf (27, 28) oder in der Verdünnungsleitung (31) strömenden Flüssigkeit abhängig von einem gemessenen Wert einer chemischen-physikalischen Eigenschaft;
Steuerung mindestens einer zweiten Durchfluss einer im ersten injektionskreislauf (27, 28) oder in der Verdünnungsleitung (31) strömenden Flüssigkeit abhängig von einem vorgegebenen Flusswert.

11. Verfahren nach Anspruch 10, worin die verdünnungsflüssigkeit von einer Verzweigungsstelle (32) in der Hauptleitung (26) entfernt wird und seine lonkonzentration zwischen der Verzweigungsstelle (32) und der Verdünnungsstelle (33) nicht ändert.

12. Verfahren nach Anspruch 10 oder 11, worin der erste Fluss in einem Kreislaufabschnitt strömt, der in der Gruppe umfassend die folgenden Kreislaufabschnitte ausgewählt wird: einen Kreislaufabschnitt zwischen einer Quelle für das erste Konzentrat (28) und der Verdünnungsstelle (33); einen Kreislaufabschnitt zwischen der Verdünnungsstelle (33) und der ersten Injektionsstelle (29); und einen Kreislaufabschnitt aufwärts von der Verdünnungsstelle (33), wo die Verdünnungsflüssigkeit strömt; wobei der zweite Fluss in einem der oben genannten Kreislaufabschnitte strömt, aber in einem anderen Abschnitt als der Abschnitt, wo der erste Fluss strömt.

13. Verfahren nach irgendeinem der Ansprüche 10 bis 12, worin die Hauptleitung (26) eine erste Flüssigkeit, z.B. Wasser, aus einer Flüssigkeitsquelle (24) empfängt und eine zweite Flüssigkeit, z.B. eine Dialyseflüssigkeit, zu einer Blutbehandlungsvorrichtung (2) sendet.

14. Verfahren nach irgendeinem der Ansprüche 10 bis 13, worin die chemische-physikalische Eigenschaft die elektrische Leitfähigkeit oder eine andere die Ionkonzentration angebende Eigenschaft ist.

## Revendications

1. Appareil pour la préparation en ligne d'un liquide médical, comprenant:
une ligne principale (26) pour la préparation du liquide médical, reliée à une entrée (24) d'un liquide et ayant au moins un premier point d'injection (29) d'un concentré;
un premier circuit d'injection (27, 28) relié à une entrée d'un premier concentré (28) et au premier point d'injection (29),
un premier capteur (30) apte à émettre un signal indiquant une propriété chimique-physique du liquide présent dans le premier circuit d'injection (27),
une unité de commande (22) apte à commander une première pompe (34) en fonction du signal émis par le premier capteur (30), **caractérisé en ce que**
le premier circuit d'injection a un point de dilution (33) situé entre l'entrée du premier concentré (28) et le premier point d'injection (29); et **caractérisé en ce que** l'appareil comprend en outre:
une ligne de dilution (31) portant un liquide de dilution au point de dilution (33) où le premier concentré est dilué;
la première pompe (34) et une deuxième pompe (35) pour la circulation de liquides dans le premier circuit d'injection (27, 28) et dans la ligne de dilution (31); le premier capteur (30) étant apte à émettre un signal indiquant une propriété chimique-physique du liquide situé dans le premier circuit d'injection (27) entre le point de dilution (33) et le premier point d'injection (29).

2. Appareil selon la revendication 1, où l'unité de commande (22) est apte à commander la deuxième pompe (35) en fonction d'une valeur indiquant un débit prédéfini.

3. Appareil selon la revendication 2, comprenant un deuxième capteur étant à même d'émettre un signal indiquant un débit de fluide dans une portion où la deuxième pompe (35) est opérationnelle, l'unité de commande (22) étant apte à commander la deuxième pompe (35) en fonction du signal émis par le deuxième capteur.

4. Appareil selon la revendication 3, où le deuxième capteur comprend un capteur pour détecter une vitesse de travail de la deuxième pompe (35) ou un débitmètre pour mesurer le débit de fluide dans la section où la deuxième pompe (35) est opérationnelle.

5. Appareil selon une quelconque des revendications précédentes, où la première pompe (34) est positionnée dans un endroit choisi dans le groupe comprenant les trois endroits suivants: entre l'entrée du premier concentré (28) et le point de dilution (33), entre le point de dilution (33) et le premier point d'injection (29), et dans la ligne de dilution (31); la deuxième pompe (35) étant positionnée dans un des trois endroits sus-mentionnés mais dans un endroit différent par rapport à la première pompe (34).

6. Appareil selon une quelconque des revendications précédentes, où la ligne de dilution (31) a une extrémité d'entrée reliée à un point de branchement (32) situé dans la ligne principale (26) en amont du premier pompe d'injection (29), par rapport à une direction de circulation du liquide dans la ligne principale (26), et est apte à ne pas induire de grands changements d'une concentration ionique du liquide de dilution s'écoulant de l'extrémité d'entrée (32) au point de dilution (33).

7. Appareil selon une quelconque des revendications précédentes, où la ligne principale (26) s'étend entre l'entrée (24) d'un liquide, par exemple eau, et un raccord pour la liaison à un dispositif de traitement de sang (2), par exemple un dialyseur.

8. Appareil selon une quelconque des revendications précédentes, où le premier capteur (30) comprend au moins un senseur indiquant une concentration ionique d'un liquide, tel que par exemple un capteur de conductivité électrique.

9. Machine pour le traitement extracorporel de sang, comprenant un appareil pour la préparation en ligne d'un liquide médical (23), réalisé selon une quelconque des revendications précédentes.

10. Procédé pour la préparation en ligne d'un liquide médical, comprenant les étapes suivantes:
faire circuler un liquide dans une ligne de préparation principale (26) ayant au moins un premier point d'injection (29) d'un concentré;
injecter au moins un premier concentré d'un premier circuit d'injection (27, 28) au premier point d'injection (29); **caractérisé en ce que** il comprend en outre les étapes suivantes:
injecter un liquide de dilution d'une ligne de dilution (31) à un point de dilution (33) du premier circuit d'injection (27) pour réduire une concentration ionique du premier concentré avant d'atteindre le premier point d'injection (29);
mesurer au moins une valeur d'une propriété chimique-physique du premier concentré dilué s'écoulant dans le premier circuit d'injection (27, 28) après le point de dilution (33);
commander au moins un premier débit d'un liquide s'écoulant dans le premier circuit d'injection (27, 28) ou dans la ligne de dilution (31) en fonction d'une valeur mesurée d'une propriété chimique-physique;
commander au moins un deuxième débit d'un liquide s'écoulant dans le premier circuit d'injection (27, 28) ou dans la ligne de dilution (31) en fonction d'une valeur de débit prédéfinie.

11. Procédé selon la revendication 10, où le liquide de dilution est éliminé d'un point de branchement (32) dans la ligne principale (26) et ne modifie pas sa concentration ionique entre le point de branchement (32) et le point de dilution (33).

12. Procédé selon la revendication 10 ou 11, où le premier flux s'écoule dans une portion de circuit choisie dans le groupe comprenant les portions de circuit suivantes: une portion de circuit entre une source du premier concentré (28) et le point de dilution (33); une portion de circuit entre le point de dilution (33) et le premier point d'injection (29); et une portion de circuit en amont du point de dilution (33) où le liquide de dilution s'écoule; le deuxième flux s'écoulant dans une des portions sus-mentionnées mais dans une portion différente de la portion où s'écoule le premier flux.

13. Procédé selon une quelconque des revendications 10 à 12, où la ligne principale (26) reçoit un premier liquide, par exemple eau, d'une source de liquide (24), et envoie un deuxième liquide, par exemple un liquide de dialyse, à un dispositif de traitement de sang (2).

14. Procédé selon une quelconque des revendications 10 à 13, où la propriété chimique-physique est la conductivité électrique ou une autre propriété indiquant la concentration ionique.
